(19) Europäisches Patentamt
European Patent Office
Office européen des brevets

(11) Publication number: 0 354 714 A2

(12) EUROPEAN PATENT APPLICATION

(21) Application number: 89307825.3

(22) Date of filing: 01.08.89

(51) Int. Cl.⁴: A61K 31/135 , A61K 31/655 , A61K 31/19

(30) Priority: 12.08.88 IL 87444
16.07.89 IL 90993

(43) Date of publication of application:
14.02.90 Bulletin 90/07

(84) Designated Contracting States:
AT BE CH DE ES FR GB GR IT LI LU NL SE

(71) Applicant: HADASSAH MEDICAL ORGANIZATION
Kiryat Hadassah P.O. Box 12000
IL-91120 Jerusalem(IL)

(72) Inventor: Ben-Sasson, Shmuel
10 Kiryat Moshe Street
Jerusalem(IL)
Inventor: Eilat, Dan
20 Burla Street
Jerusalem(IL)

(74) Representative: Goodanew, Martin Eric et al
MATHISEN, MACARA & CO. The Coach House 6-8 Swakeleys Road
Ickenham Uxbridge UB10 8BZ(GB)

# (54) Pharmaceutical compositions containing polyaromatic compounds.

(57) The invention provides a pharmaceutical composition for affecting tissue redistribution of bioactive peptides and proteins which are normally bound to glycosaminoglycans, and for mimicking the action of glycosaminoglycans in biological interactions, comprising an active ingredient in combination with a pharmaceutically acceptable carrier, the active ingredient being a therapeutically effective amount of a pharmaceutically acceptable polymeric compound having monomeric units and a molecular weight between 1,000 and 20,000 Daltons and pharmaceutically acceptable salts and esters thereof, wherein each monomeric unit contains between 3 and about 10 aromatic rings.

EP 0 354 714 A2

# GAG MIMIC

The present invention relates to pharmaceutical compositions containing polyaromatic compounds as an active ingredient therein.

More particularly, the present invention relates to a pharmaceutical composition for affecting tissue redistribution of bioactive peptides and proteins normally bound to glycosaminoglycans and for mimicking the action of glycosaminoglycans in various biological interactions.

The term "tissue redistribution" as used herein implies either a change in the compartmentalization of a constituent within a given tissue or a change in the distribution pattern between different tissues. For example, the release of a peptide from a basement membrane which can result in its consumption by surrounding cells or its transfer into the blood stream. Alternatively, it can be exemplified by a competitive binding of a soluble compound to a protein, thereby preventing its association with certain tissue-fixed residues.

As is known, glycosaminoglycans (acid mucopolysaccharides) are a major constituent participating in the composition of various biological structures such as basement membranes, connective tissues, cartilage and cell-surface glycocalyx.

Connective tissues are responsible for providing and maintaining form in the body. Functioning in a mechanical role, they provide a matrix that serves to connect and bind the cells and organs and ultimately give support to the body. Unlike the other tissue tyres (epithelium, muscle, and nerve) formed mainly by cells, the major constituent of connective tissue is its extracellular matrix, composed of protein fibers, an amorphous ground substance, and tissue fluid, the latter consisting primarily of bound water of solvation. Embedded within the extracellular matrix are the connective tissue cells.

In terms of structural composition, connective tissue can be subdivided into three classes of components: cells, fibers, and ground substance. The wide variety of connective tissue types in the body represents modulations in the degree of expression of these three components.

The amorphous intercellular ground substance fills the space between cells and fibers of the connective tissue; it is viscous and acts as a lubricant and also as a barrier to the penetration of the tissues by foreign particles. The ground substance is formed mainly by two classes of components: glycosaminoglycans and structural glycoproteins.

Glycosaminoglycans (GAG) are linear polysaccharides formed by characteristic repeating disaccharide units usually composed of a uronic acid and a hexosamine. The term "acid mucopolysaccharides" was used originally to designate hexosamine-rich acid polysaccharides extracted from connective tissue. In recent years, the term "glycosaminoglycans" has gained greater acceptance and is now used in place of mucopolysaccharides. The hexosamine can be glucosamine or galactosamine, and the uronic acid can be glucuronic or iduronic acid. Sulphate groups are found on all glycosaminoglycans apart from hyaluronic acid, and all of the sulphated glycosaminoglycans are covalently linked to protein forming different classes of proteoglycans. However, it would be an oversimplification to consider glycosaminoglycans to be simple repeat-unit polysaccharides, since considerable chemical and configurational variability can be superimposed upon the component sugars.

Among other functions it has been shown that the glycosaminoglycans serve also as a support which binds various bioactive peptides. This association is based on a non-covalent interaction since the bound protein can be readily released upon the addition of free glycosaminoglycans such as heparin. Well known examples include enzymes such as lipoprotein lipase (LPL) or growth-regulating peptides such as fibroblast growth factor (FGF). In addition, it has been demonstrated that heparin inhibits the growth of vascular smooth muscle cells and the proliferation of kidney mesangial cells. The former cell type is involved in atherosclerosis while the latter plays a role in glomerulosclerosis. Another example of GAG-protein interaction is that of the enzyme heparanase which participates in cell-invasion processes.

According to the invention, there is provided a pharmaceutical composition comprising, an active ingredient in combination with a pharmacologically acceptable carrier, the active ingredient being a therapeutically effective amount of a pharmaceutically acceptable polymeric compound having monomer units wherein each monomeric unit contains between about three and ten aromatic rings. The ring can contain electro-negative substituents and/or negatively charged residues on at least two of the rings.

With the present invention it has been realized that the ability to compete with the binding of bioactive peptides to glycosaminoglycans, via the application of synthetic, biocompatible molecules, opens the way for therapeutical manipulations of many important systems.

The invention makes use of a family of polymeric compounds which can modulate biological systems containing complexes between bioactive peptides (proteins) and glycosaminoglycans and can mimic the

action of glycosaminoglycans in various biological interactions.

Pharmaceutical compositions containing these compounds can be used for:

1. Removal of cationic proteins from the glomerular basement membrane or connective-tissues, thereby preventing local damage (i.e., via "recharging" of negatively charged residues in the glomerular basement membrane).

2. Modulation of LPL, a key enzyme in lipid distribution among various tissues which could be implicated in cardiovascular diseases.

3. Release of growth-promoting molecules, such as fibroblast growth factor (FGF), from basement membranes in order to enhance the process of angiogenesis and wound-healing. In addition, FGF can prevent death of lesioned neurons (see Anderson K.J. et al. Nature 322:360-1 (1988). Therefore, the release of endogenously-stored FGF might be beneficial for the treatment of neuronal disorders such as Alzheimer's disease and other dementia.

4. Blocking the activity of heparanase, an enzyme which participates in inflammatory processes and metastases formation.

5. Modulation of bone metabolism.

6. Control of the proliferation of certain cell types such as smooth muscle cells or mesangial cells.

For example, heparin is a commercially available glycosaminoglycan useful in the release of lipoprotein lipase, the inhibition of heparanase or the release of fibroblast growth factor. The most common application of heparin is as an anticoagulant where this GAG molecule interacts with proteins which play a key role in hemostasis. Thus, in another aspect of the present invention, pharmaceutical compositions of the present invention can be used as anticoagulants.

Preferrred polymeric compounds used in the pharmaceutical compositions of the present invention are those wherein each monomeric unit contains between three and ten aromatic rings and have one or more substituent(s) on at least two of the aromatic rings. The substituents include -NRR$_1$, -N=R, -OR, =0, -$N0_2$, -COOR, halogen, -$S0_2$OR, -$SO_2$NHR, -0$S0_2$OR, and -R, wherein R is lower alkyl or hydrogen and $R_1$ is lower alkyl, hydrogen or substituted phenyl.

The polymeric compounds useful in the pharmaceutical composition of the present invention include the pharmaceutically acceptable salts or esters of the compounds. The compound can be a salt with a cation such as sodium, potassium or ammonia or in compounds wherein the substituent is -C00R, said compound can be a $C_1$-$C_{12}$ ester.

The preferred number of aromatic rings in each monomeric unit is between three and six.

The molecular weight of the polymeric compounds is between 1,000 and 20,000, more preferably between 2,000 and 4,000 Daltons, determined by gel permeation chromatography.

The following gel permeation chromatography conditions were utilized to determine the molecular weights of compounds containing aurintricarboxylic acid monomer (aluminon or ATA).

Column: Two I-125 (Waters, TN) protein analysis column in series

Mobile Phase: Water (70%)-propanol (30%) containing 0.1% (V/V) trifluoroacetic acid

Detection: UV at 220mm

Injection: Sample was dissolved in the mobile phase and injected

Polymers of many known synthetic dyes having the above defined monomeric features can be used in the pharmaceutical compositions of the present invention. The known uses of those dyes include color additives, diagnostic aids administered by an i.v. injection, antiseptic agents or as a remedy for infectious diseases. Nevertheless, none of these dyes has previously been described or used as a therapeutic agent which replace glycosaminoglycans.

These synthetic dyes include Aluminon, Anazoline Sodium, Eosine I Bluish, Eosine Yellowish, Erythrosine, Evan's Blue, Fast Green FCF, Fucshin(e) Acid, Iodophthalein Sodium, Rose Bengal, Sulfobromophthalein Sodium, Suramin Sodium, Trypan Blue, Trypan Red, Rosaniline Chloride, Crystal Violet, Methyl Blue, Methyl Green, Coomassie Blue, Basic Fuchsin, Malachite Green, Brilliant Green, Aniline Blue, Brilliant Cresyl Blue, Safranin O, Ethyl Violet, Pararosaniline Acetate, Methyl Violet, Halogenated Aluminon, Sulfonated Aluminon, and Sulfonated-Halogenated Aluminon. Halogenated, Sulfonated and Sulfonated-Halogenated Aluminon is Aluminon in which a halogen replaces one or more of the hydrogens of the aromatic rings and/or sulfonate residue(s) replace one or more of the carboxylic groups.

More preferred is a polymeric compound of the structure:

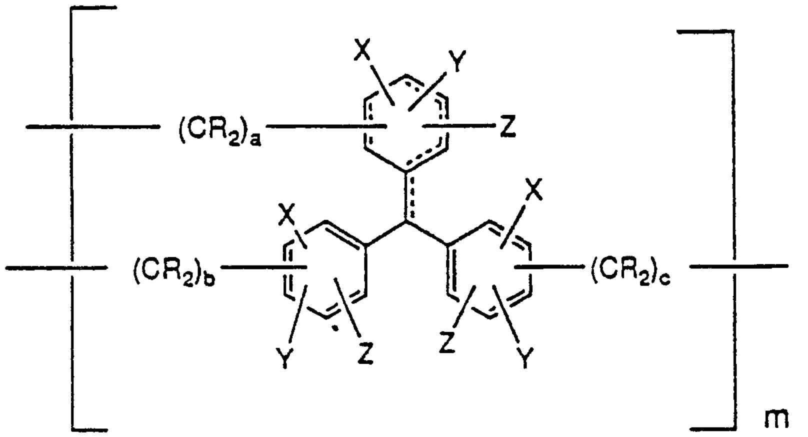

or a salt or ester thereof, wherein a, b, and c are independently 0 or 1, m is 5 to 20, dashed lines represent single or double bonds, each aromatic ring is substituted with at least one of X, Y, and Z independently selected from $-NRR_1$, $-N=R$, -OR, =0, $-NO_2$, -COOR, halogen, $-SO_2OR$, $-SO_2NHR$, $-OSO_2OR$, and -R, wherein R is lower alkyl or hydrogen and $R_1$ is lower alkyl, hydrogen or substituted phenyl, and the polymerization is through carbon linkages.

Even more preferred is a polymeric compound of the structure:

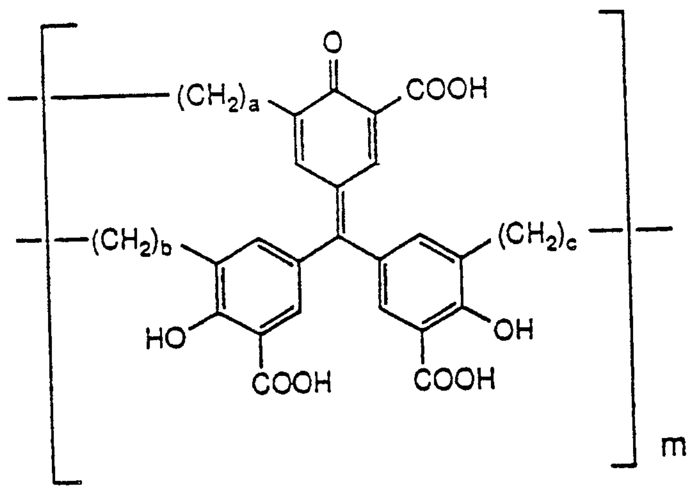

wherein a, b and c are independently 0 or 1, wherein $a+b+c \geqq 2$, and m is 5 to 20.

While the invention will now be described in connection with certain preferred embodiments in the following examples so that aspects thereof may be more fully understood and appreciated, it is not intended to limit the invention to these particular embodiments. On the contrary, it is intended to cover all alternatives, modifications and equivalents as may be included within the scope of the invention as defined by the appended claims. Thus, the following examples which include preferred embodiments will serve to illustrate the practice of this invention, it being understood that the particulars shown are by way of example and for purposes of illustrative discussion of preferred embodiments of the present invention only and are presented in the cause of providing what is believed to be the most useful and readily understood description of formulation procedures as well as of the principles of conceptual aspects of the invention.

EXAMPLE 1:

The following comparative compounds demonstrate that the pharmaceutical activity of commercially available aluminon (aurintricarboxylic acid or ATA) is produced by the fraction containing polymers of monomeric units of ATA and having a molecular weight of 2,000-20,000 Daltons.

## Experimental Procedures

### Comparative Compound A

- Commercially available aurin tricarboxylic acid (ATA) from Aldrich Chemical Co., Wisconsin

### Comparative Compound B

- Polymer containing ATA monomer and having molecular weight of 2,000-4,000 Daltons

Commercially available aurintricarboxylic acid was fractionated on Biogel P-6 by elution with 50% aqueous ethanol with 50mM Tris at pH 7.5 and then 50% aqueous ethanol with 50mM glycine at pH 11. The molecular weight of the fraction as determined by the above description of gel permeation chromatography containing the greatest anti-coagulant activity was determined to be between 2,000-4,000 Daltons.

### Comparative Compound C

- ATA not containing polymers

Following the procedure described in Organic Synthesis Coll. Vol. I, page 54, the following reaction was performed to produce non-polymerized ATA. To 17 ml of concentrated sulfuric acid at 0-5 °C and mechanically stirred was added portionwise sodium nitrite (2.5 gm, 36.2 mmol). The mixture was stirred until a solution was obtained. Salicylic acid (5.0 gm, 36.5 mmol) was added portionwise over 10-15 minutes and the reaction warmed to room temperature, stirred until a homogeneous mixture was obtained and cooled to 0-5 °C. A solution of formaldehyde (formalin, 35-40%, 1.25 mL, 16.3 mmol) was added dropwise over 20 minutes. The reaction was quenched with the slow addition of 25 gm crushed ice and then 125 mL of ice water. The resulting solid was filtered, washed with water, and dissolved in dilute aqueous ammonium hydroxide. Removal of the volatiles at 90 °C and then *in vacuo* provided the red-brown solid.

### Comparative Compound D

- Polymer containing ATA monomeric units

Using the above procedure, the following reagents were utilized to produce a polymer containing ATA monomer units:
concentrated sulfuric acid: 17mL
sodium nitrite: 2.5 gm, 36.2 mmol
salicyclic acid: 5.0 gm, 36.5 mmol
formalin: 35-40%, 6.0 mL, 81.5 mmol

### Measurement of Activated Partial Thromboplastin Time (APTT)

Utilizing the following procedure, the activated partial thromboplastin time (APTT) was measured for each of comparative compounds A-D:

To an assay cuvette was added 100 ul of normal pooled plasma (George King Biomedical Inc., Kansas) and 100 ul of a solution containing one of the above compounds in aqueous 50 mM Tris hydrochloride at pH 7.5 (0.2 mg of sample in one mL buffer). The sample was placed in a MLA coagulation timer which automatically maintained the sample at 37 °C for 2.5 minutes, injected 100 ul of actin activated cephaloplastin reagent, kept 5 minutes, injected 100 ul of 3 mM CaCl, and the clot formation was determined photometrically and the time recorded. The results are as follows:

Table I.

| | Compound | Concentration (mg/mL) | APTT (sec.) |
|---|---|---|---|
| | Normal pooled plasma | ----- | 25 - 28 |
| A - | Commercial ATA | 0.2 | 40 - 60 |
| C - | Non-Polymerized ATA | 0.2 | 28 |
| D - | Polymer containing ATA monomer | 0.2 | 72 |
| B - | Polymer having molecular weight of of 2,000-4,000 containing ATA monomer | 0.2 | 110 |

All the following examples use commercially available compounds. Based on the above example, the activity is believed to be due to the polymeric fractions of the commercially available products.

EXAMPLE 2:

Modulation of lipoprotein lipase (LPL) levels and compartmentalization

The enzyme LPL participate in the process of lipid transfer from the bloodstream to the tissues, LPL is bound to the external surface of cells via its association with cell-membrane glycosaminoglycans. Cultured heart cells is a powerful system in the study of the enzyme turn-over and the exploration of ways to manipulate its level since these cells are active in the biosynthesis of LPL, in addition to the surface binding of the enzyme.

The following are the materials and methods used:

F· heart cell cultures were prepared from newborn rat hearts as previously described (Chajek, T. et al., Biochem. Biophys. Acta 528, 456-474 (1978)). They consisted mainly of non-beating mesenchymal cells and were used 8-12 days after their isolation.

The enzyme activity was determined on aliquots of medium and of homogenates of cells which had been released from the petri dish with a rubber policeman in 1 ml of 0.025 M $NH_3/NH_4Cl$ buffer (pH 8.1) containing 1 unit/ml of heparin. The assay system consisted of 0.1 ml of medium or 0.1 ml of cell homogenate (50-70 g protein) and 0.1 ml of substrate containing labeled triolein, prepared according to the method of Nilsson-Ehle and Schotz, (J. Lipid Res. 17, 536-541 (1976)). Incubations were carried out at 37° C for 45 min. The reaction was stopped by the addition of methanol/chloroform/heptane (1.4:1.25:1.v/v) and the extraction of fatty acids was performed according to the method of Belfrage and Vaughan (J. Lipid Res. 10, 341-344 (1969), as modified by Nilsson-Ehle and Schotz. Enzyme activity was calculated according to the formula of Nilsson-Ehle and Schotz.

Table II demonstrates the ability to modulate (shift-up) the enzyme level by commercially available Evan's Blue and to influence its compartmentalization. Similar results were obtained with commercially available Methyl Blue, Trypan Blue and Aluminon.

Table II.

| LPL activity following 24 hrs. incubation of heart cells with commercially available Evan's Blue LPL activity: m mole FFA/h/dish (mean±SEM of triplicates) | | | | |
|---|---|---|---|---|
| Evan's Blue Concentration (M) | Cells | Medium | Total | % increase in the total |
| 0 | 2420±93 | 817±14 | 3237 | -(0) |
| $10^{-5}$ | 2757±114 | 1531±182 | 4288 | +32.5 |
| $10^{-4}$ | 2447±48 | 2783±194 | 5230 | +61.6 |
| $10^{-3}$ | 1698±184 | 6051±435 | 7749 | +139.4 |

EXAMPLE 3:

Inhibition of heparanase activity

Heparanase is an endoglucuronidase capable of degrading heparan sulfate (HS) at specific intrachain sites. Studies on degradation of sulfated proteoglycans in the subendothelial extracellular matrix (ECM) demonstrated a correlation between heparanase activity and the metastatic potential of various tumor cells. Heparanase activity was also suggested to play a role in the mobilization of normal circulating cells of the immune system during inflammatory processes.

The ability of several of the above mentioned compounds to inhibit lymphoma-cell derived heparanse was tested in the assay system described by Vlodavsky et al. (Cancer Research. 43: 2704-2711 (1983). $^{35}S$ labled ECM was incubated for 24 hours with ESb mouse lymphoma heparanase in the presence of 10 ugr ml of the indicated compounds. Degradation of the sulfated glycosaminoglycan was followed by gel filtration of the supernatants. Heparanase activity is expressed as the total amount of labled low-molecular-weight fragments released from the ECM substrate. The results are summarized in Table III.

Table III.

| Inhibition of heparanase activity by various commercially available compounds | | |
|---|---|---|
| Compound (10 μgr ml) | Total low-mol-wt. HS fragments released by heparanase (cpm) | % Inhibition |
| Control | 17,756 | -- |
| Methyl Blue | 13,676 | 23 |
| Fast Green | 12,139 | 32 |
| Evan's Blue | 6,986 | 60 |
| Aluminon | 3,822 | 78 |

EXAMPLE 4:

Anticoagulant effect

The anticoagulant effect of commercially available aluminon was demonstrated by measuring the partial thromboplastin time (PTT) in blood samples from a normal donor. 0.25 ml of the indicated concentration of the drug (x10, diluated in saline) were added to 2.25 ml of fresh human blood, collected in the presence of citrate. PTT was measured following the addition of $Ca^{++}$. The results are summarized in the following Table IV.

Table IV.

| PTT of human blood in the presence of various concentrations of commercial available aluminon | |
|---|---|
| Aluminon final conc. (mg/ml) | PTT (sec.) |
| 1 | >300 |
| 0.1 | 190 |
| 0.01 | 30.5 |
| 0 (control) | 30.5 |

As can be seen, commercial available aluminon is an effective anticoagulant. Moreover, since aluminon can be absorbed through the gastrointestinal tract, it can be used as an orally administratable anticoagulant (as opposed to heparin which has to be given by injection). Indeed, an oral administration of commercial available aluminon to rats (1 ml of 10%-20% solution) prolonged their PTT considerably (from around 20 sec. at time 0 to >200 sec. after 2-4 hours); concomitantly, plasma levels of LPL were elevated by about an order of magnitude.

EXAMPLE 5:

Inhibition of the proliferation of vascular-smooth muscle cells

Bovine aortal smooth-muscle cells were grown in tissue culture plates in the presence of glucose-rich medium (H-21). The cultures (around $35 \times 10^3$ cells/well in 24 wells' plates) were initially supplemented with 10% fetal calf serum (FCS) for 2-3 days. The cell nutrition was then replaced by a serum-deprived medium (H-21 plus 0.2% FCS) in order to test the effect of defined growth factors on the proliferation of smooth-muscle cells and their possible inhibition by the compounds with heparin-like activity.

In the following representative experiment, the cells transferred to the serum-deprived medium were grown in the presence of thrombin ($10^{-6}$ M) or FGF (partially purified from bovine brain, 250 ngr/ml). Various concentrations of aluminon were added to the thrombin or FGF-treated cells and to non-treated control cultures. $^3$H thymidine was also added to all wells for an incubation period of 2 days. The cultures were then harvested and the $^3$H thymidine incorporated into DNA was measured. The results are shown in Table 5.

Table V.

| Growth inhibition of resting, FGF or thrombin-stimulated smooth muscle cells by commercially available aluminon | | | |
|---|---|---|---|
| Aluminon Concentration (µgr/ml) | $H^3$ thymidine incorporation per well (cpm. mean of duplicates) | | |
| | Control | +FGF | +Thrombin |
| 0 | 9.823 | 22,574 | 54,000 |
| 0.2 | 8,425 | 22,256 | 46,857 |
| 2 | 5,120 | 13,089 | 14,064 |
| 10 | 4,105 | 6,846 | 6,877 |
| 20 | 4,343 | 6,745 | 5,705 |

More than 40% inhibition is already achieved at aluminon concentration of 2 µgr/ml (~$4x10^{-6}$ M).

In other words, the compound can mimic the action of heparin also in this system as a potent inhibitor of the proliferation of vascular smooth muscle cells.

EXAMPLE 6:

The polymeric components of the following dyes, at the concentration listed, significantly prolonged the activated partial thromboplastin time when compared to a standard buffer solution.

| Compound | Minimum Final Concentration (ug/ml) |
|---|---|
| Rosaniline Chloride | 12.5 |
| Crystal Violet | 8.3 |
| Methyl Blue | 100.0 |
| Methyl Green | 50.0 |
| Coomassie Blue | 150.0 |
| Basic Fuchsin | 50.0 |
| Malachite Green | 150.0 |
| Brilliant Green | 150.0 |
| Aniline Blue | 150.0 |
| Brilliant Cresyl Blue | 150.0 |
| Safranin O | 150.0 |
| Ethyl Violet | 50.0 |
| Pararosaniline Acetate | 150.0 |
| Methyl Violet | 75.0 |

It will be evident to those skilled in the art that the invention is not limited to the details of the foregoing illustrative examples and that the present invention may be embodied in other specific forms without departing from the essential attributes thereof, and it is therefore desired that the present embodiments and examples be considered in all respects as illustrative and not restrictive, reference being made to the appended claims, rather than to the foregoing description, and all changes which come within the meaning and range of equivalency of the claims are therefore intended to be embraced therein.

## Claims

1. A pharmaceutical composition for affecting tissue redistribution of bioactive peptides and proteins which are normally bound to glycosaminoglycans, and for mimicking the action of glycosaminoglycans in biological interactions, comprising an active ingredient in combination with a pharmaceutically acceptable carrier, the active ingredient being a therapeutically effective amount of a pharmaceutically acceptable polymeric compound having monomeric units and a molecular weight between 1,000 and 20,000 Daltons and pharmaceutically acceptable salts and esters thereof, wherein each monomeric unit contains between about 3 and about 10 aromatic rings.

2. A pharmaceutical composition according to claim 1 wherein said aromatic rings contain at least one substituent on at least two of the rings.

3. A pharmaceutical composition of claim 2 wherein the substituents are selected from $-NNR_1$, $-N=R$, $-OR$, $=O$, $-NO_2$, $-COOR$, halogen, $-SO_2OR$, $-SO_2NHR$, $-OSO_2OR$, and $-R$, wherein R is lower alkyl or hydrogen, and $R_1$ is lower alkyl, hydrogen, or substituted phenyl.

4. A pharmaceutical composition according to claim 1 wherein the active ingredient has anticoagulant properties.

5. A pharmaceutical composition according to claim 1 administered orally.

6. A pharmaceutical composition according to claim 1 wherein the active ingredient is absorbed in the gastrointestinal tract.

7. A pharmaceutical composition according to claim 1 wherein said monomer contains between 3 and 6 aromatic rings.

8. A pharmaceutical composition according to claim 1 wherein said polymer contains monomer units selected from Aluminon, Halogenated Aluminon, Sulfonated Aluminon, Sulfonated-Halogenated Aluminon, Anazolene Sodium, Eosine I Bluish, Eosine Yellowish, Erythrosine, Evan's Blue, Fast Green FCF, Fucshin(e) Acid, Iodophthalein Sodium, Methyl Blue, Rose Bengal, Sulfobromophthalein Sodium, Suramin Sodium, Trypan Blue, Trypan Red, Rosaniline Chloride, Crystal Violet, Methyl Green, Coomassie Blue, Basic Fuchsin, Malachite Green, Brilliant Green, Aniline Blue, Brilliant Cresyl Blue, Safranin O, Ethyl Violet, Pararosaniline Acetate, and Methyl Violet.

9. A pharmaceutical composition comprising an active ingredient in combination with a pharmaceutically acceptable carrier, the active ingredient being a polymer of the following structure:

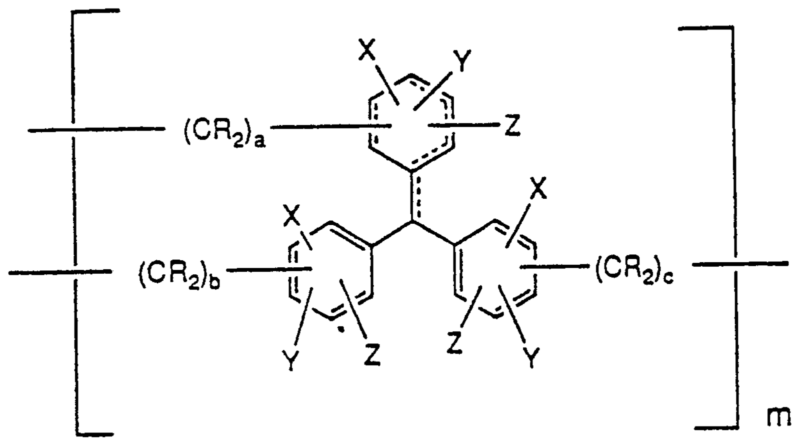

or a salt or ester thereof, wherein a, b, and c are independently 0 or 1, m is 5 to 20, dashed lines represent single or double bonds, each aromatic ring is substituted with at least one of X, Y, and Z independently selected from NRR$_1$, -N=R, -OR, =0, -N0$_2$, -COOR, halogen, -S0$_2$OR, -SO$_2$NHR, -0S0$_2$OR, and -R, wherein R is lower alkyl or hydrogen and R$_1$ is lower alkyl, hydrogen or substituted phenyl, and the polymerization is through carbon linkages.

10. A pharmaceutical composition comprising an active ingredient in combination with a pharmaceutically acceptable carrier, the active ingredient being a polymer of the following structure:

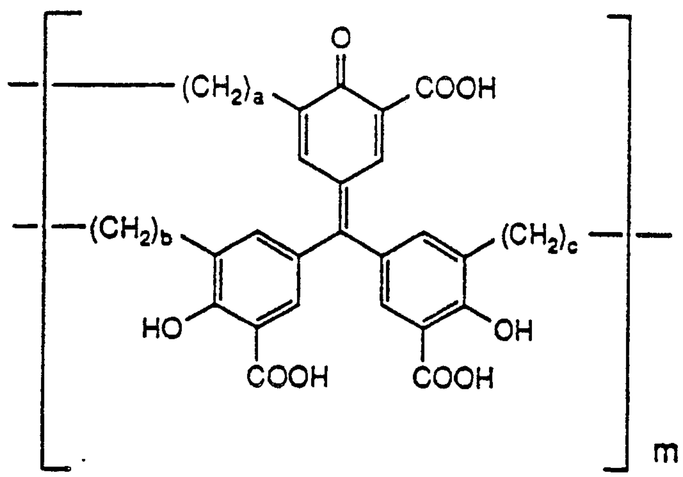

or a salt or ester thereof, wherein a, b, and c are 0 or 1 and $a+b+c \geqq 2$, and m is 5-20.

11. A pharmaceutical composition of claim 10 wherein the molecular weight of the polymer is between 2,000 and 4,000 Daltons.

12. The pharmaceutical composition of claim 10 in which the active ingredient is absorbed in the gastrointestinal tract and has anticoagulent properties.

13. The use of a therapeutically effective amount of a pharmaceutically acceptable polymeric compound, or a salt or ester thereof, the polymeric compound having a molecular weight of between 1,000 and 20,000 Daltons and containing monomeric units, wherein each monomer unit contains between 3 and 10 aromatic rings and has at least one substituent on at least two of the monomer rings, for the manufacture of a medicament for affecting tissue redistribution of bioactive peptides and proteins normally bound to glycosaminoglycans, and for mimicking the action of glycosaminoglycans in biological interactions.

14. A method for affecting tissue redistribution of bioactive peptides and proteins which are normally bound to glycosaminoglycans and for mimicking the action of glycosaminoglycans in biological interactions, comprising administering as an active ingredient in combination with a pharmaceutically acceptable carrier, a therapeutically effective amount of a pharmaceutically acceptable polymeric compound, or a salt or ester thereof, the polymeric compound having a molecular weight of between 1,000 and 20,000 Daltons and containing monomeric units, wherein each monomer unit contains between 3 and 10 aromatic rings and have at least one substituent on at least two of the monomer rings.